# EUROPEAN PATENT SPECIFICATION

(11) **EP 1 019 024 B1**
(45) Date of publication and mention of the grant of the patent: **07.01.2004**
(21) Application number: 98949592.4
(22) Date of filing: 30.09.1998
(51) Int. Cl.: A61K 9/12, A61K 9/70

(54) **AEROSOL OINTMENT COMPOSITIONS**
AEROSOL-SALBEN-ZUBEREITUNGEN
COMPOSITIONS AEROSOL COMPRENANT UN ONGUENT

(30) Priority: 02.10.1997 US 947530; 08.05.1998 US 75067
(43) Date of publication of application: 19.07.2000
(73) Proprietor: OMS Holdings, LLC, New York, NY 10154 (US)
(72) Inventor: OSIPOW, Lloyd, I., Boynton Beach, FL 33437 (US); MARRA, Dorothea, C., Summit, NJ 07901 (US); SPITZER, Joseph, George, Palm Beach, FL 33480 (US)
(74) Representative: Campbell, Neil Boyd
(86) International application number: PCT/US1998/020335
(87) International publication number: WO 1999/017739

(56) References cited:
- WO-A-92/11839
- WO-A-97/10802
- DE-A- 3 810 730
- GB-A- 1 123 868
- GB-A- 1 527 061
- US-A- 3 947 566
- US-A- 4 127 663
- US-A- 5 457 128
- US-A- 5 618 515

## Description

### Background of the Invention

U.S. Patent No. 4,422,877 to Spitzer et al discloses aerosol synthetic polymer - liquefied propellant compositions which when expelled from an aerosol container form cold pad polymeric foamed structures whose temperature is initially at least 30°C below the ambient temperature at which the cold formed structure is formed, said formed structures containing open and/or closed cells which may contain an additive which is deposited in the pores and/or walls of the foamed structure as the foamed structure is formed. The aforedescribed aerosol compositions prior art when expelled on a surface exert a pronounced cooling effect on said surface until the propellant component thereof is completely evaporated.

The essential ingredients of the aerosol compositions of the above-mentioned U.S. Patent No. 4,422,877 are:
a. a film-forming synthetic polymer in an amount within the range from about 2% to about 30% by weight of the composition;
b. at least one liquefied propellant boiling below -10°C;
c. the total propellant being in an amount within the range from about 50% to about 90% by weight of the composition; and having a heat vaporization of at least 55 calories per gram; the propellant being capable of dissolving the synthetic polymer at least in the presence of a co-solvent that is soluble in the propellant and in solutions of the synthetic polymer in the propellant at ambient temperature; and
d. at least one nonsolvent that is soluble in the propellant but in which the synthetic polymer is insoluble in an amount within the range from about 1% to about 85% by weight of the composition;
the composition forming on volatilization of propellant at ambient temperature a coherent formed structure containing open and/or closed cells, and having a temperature at least 30°C below ambient temperature.

DE-A-3810730 discloses oinments which can be sprayed to yield finely distributed homogeneous mists.

### Summary of the Invention

The present invention relates to an aerosol composition consisting essentially of an ointment - liquified propellant composition which when expelled from an aerosol container onto damaged tissue provides a cold ointment which exerts a therapeutic effect on said tissue in contact therewith.

The cooling effect provided by the expelled composition of this invention is controlled so as to provide relief of pain for a desirable period of time but not too cold to cause discomfort or tissue damage.

Thus, an object of this invention is to provide a therapeutic ointment composition which when expelled from an aerosol container is cold enough to provide a cooling effect for pain relief but not too cold as to cause discomfort to damaged tissue to which the ointment is applied, said ointment also exerting a therapeutic effect on damaged tissue in contact therewith.

A further object is an ointment that can deliver appropriate medication as well as a cooling effect where it is applied.

Another object of the invention is to provide a cold ointment for the temporary relief of hemorrhoids which when applied to the swollen inflamed tissue provides a cooling effect and quickly relieving pain and itching as well as effecting shrinking of swollen inflamed tissue.

A still further object of the invention is an ointment for the treatment of sunburn.

Another object of the invention is a cold anti-itch ointment as well as one that provides relief from arthritic pain.

An additional object of the invention is to provide a cold ointment that is initially unctious, but dries to leave a deposit that is neither greasy nor oily.

More particularly, the present invention relates to novel aerosol compositions that enhance the therapeutic action of an ointment by instantly producing a sustained cooling effect which provides fast relief from pain and itching as well as a tendency to shrink swollen, inflamed tissue in advance of the slower action of any medication present in the ointment, said aerosol composition consisting essentially of from about 10 to about 60 percent by weight of an ointment component and from about 40 to about 90 percent by weight of liquefied propellant that is predominantly a non-polar propellant, and where the sum of the ointment component and the propellant equals 100 percent by weight of the composition.

The compositions used in the practice of this invention consist essentially of an ointment, i.e., a solid or semisolid component, dissolved and/or dispersed in a liquified propellant in a suitable aerosol container. The product is expelled from the aerosol container either as a deposit confined to a small area or as a spray covering a wider area, depending on the application.
Thus, to relieve hemorrhoids the deposit should be confined to a small area, while to relieve sunburn a wider area is likely to be more convenient.

The expelled therapeutic composition for this invention will feel cold due to the evaporation of the propellant. A substantial portion of the propellant that is expelled should initially be part of the deposit, so that there is a continuing cooling action as the propellant gradually evaporates. It is also important that the deposit have a comparatively high density and that it be applied thickly.

The temperature of the expelled deposit should be initially in the range of about -5°C to about +5°C. In this range the deposit can have the therapeutic effects that are the objects of this invention while not being so cold as to cause pain or tissue damage. It has now been found that n-butane is the preferred propellant for use in the compositions of this invention. n-Butane has a boiling point of -0.5°C and will tend to maintain the deposit at about that temperature. If the deposit gets much cooler, further cooling by evaporation will slow substantially. The deposit will remain in the required temperature range until the proportion of n-butane in the deposit has become quite low.

A related reason for preferring n-butane is that it has a lower vapor pressure than the more widely used liquified propellants: isobutane and propane. The lower vapor pressure assures that less propellant will be lost through evaporation as the exudate travels from the aerosol valve to the substrate upon which it is to be deposited.

However, for those products that are likely to be used at lower ambient temperatures, where n-butane does not provide sufficient pressure to expel the composition properly, it is advantageous to combine n-butane with a lesser amount of a higher vapor pressure propellant, e.g., isobutane, propane or dimethyl ether.

The distance of the spray path as well as the characteristics of the package play a role in determining how much propellant will be lost as the exudate travels to the substrate upon which it will be deposited. It is evident that the longer the spray path, the more propellant will be lost by evaporation before reaching the substrate and the less propellant will be available for sustained cooling. It has also been found that restrictions in the delivery system also promote early evaporation of propellant by reducing the flow rate of the exudate.

However, with some compositions a restricted delivery system is beneficial, since it results in a heavier-bodied deposit. Also, where layering of the composition occurs within the container, it is advantageous to employ a capillary dip tube, i.e., a dip tube with an inside diameter of 0.1 mm., to minimize the amount of separated material that is released after first shaking the container. Shaking is not effective in mixing material that is in the dip tube.

The dynamic physical characteristics of the composition play an important role in determining the amount of propellant in the deposit and the amount of time it will remain in the deposit to provide sustained cooling. The aerosol compositions of this invention consist of an ointment that generally is a dispersion of thickening agent in liquid solution of an oil, and often one or more medicinal ingredients, dispersed and/or dissolved in an appropriate propellant so that the expelled deposit is initially in the range of about -5°C to about +5°C. As product is expelled there is some loss of propellant accompanied by cooling of the exudate. If the deposit had been a liquid rather than an ointment, it would have spread rapidly whereby expiration of the propellant would occur too quickly and one would not obtain the desired sustained cooling effect. The compositions of this invention deposit as solids or semisolids. The thickness of the deposit helps to provide sustained therapeutic cooling.

It has been found that the ointment component of the composition should have a flow temperature that is at least about 35°C; otherwise, the deposit will liquefy readily and not provide sufficient cooling. It is advantageous that the flow temperature of the ointment component of the composition not exceed about 60°C, otherwise manufacture becomes more difficult.

The composition contained in an appropriate aerosol container in accordance with this invention contains from about 10 to about 60 percent by weight of an ointment with a flow temperature of at least about 35°C and from about 40 to about 90 percent by weight of a propellant that is predominantly n-butane.

### Detailed Description of the Invention

The ointment component of the composition contained in an aerosol container in accordance with the present invention includes such medically active ingredients, petroleum jellies, oils, volatile liquids, thickening agents, surfactants, and dispersed solids as may be present in the composition.

Oils that may be used in the compositions include mineral oils, silicone oils, vegetable oils such as corn oil, safflower oil, soya oil, cod liver oil, and shark liver oil and synthetic oils such as isopropyl myristate, butyl stearate and dimethyl sebacate.

Volatile organic liquids boiling below about 250°C may be used as partial or complete replacements of the oils, to provide an ointment component that dries to leave a non-greasy, non-oily residue. The polydimethylcyclosiloxanes having 3 to 5 silicone atoms are particularly useful, because of their low potential to cause irritation.

Thickening agents that may be used include mineral waxes such as paraffin and microcrystalline waxes, animal and vegetable waxes such as beeswax, wool wax, spermaceti and bayberry wax, synthetic waxes such as hydrogenated caster oil, glyceryl monostearate, cetyl palmitate and cetyl alcohol; polymers such as polyethylene and polyisobutylene and metallic soaps such as aluminum distearate.

Water may also be included in the ointment component in the form of a water -in-oil emulsion. Water is useful in a number of ways. It can act as a solvent or a dispersion medium for an active ingredient. It evaporates so that less residue remains on the skin. It reduces costs by replacing more expensive ingredients. When a portion of the aerosol composition is expelled, the deposit is a cold ointment -like structure that is a water-in-oil emulsion.

When water is included in the composition, emulsifying agents are also added to facilitate the formation of a water-in-oil emulsion. Generally, a water-soluble and an oil-soluble emulsifier are used in combination. Oil-soluble emulsifiers include the di- and tri- ethanoxy esters of lauric, myristic, palmitic and stearic acids, and the di and tri- ethanoxy ethers of lauryl alcohol, cetyl alcohol, oleyl alcohol and lanolin alcohols. Glyceryl monostearate also serves as an oil-soluble emulsifier.

Water-soluble emulsifiers include the decylethanoxy esters and ethers of the above acids and alohols, respectively; water-soluble soaps, such as potassium palmitate; anionic surfactants, such as sodium lauryl sulfate, sodium lauroyl sarcosinate and sodium stearoyl lactate; amphoteric surfactants, such as the sodium salts of the imidazoline monocarboxyl stearyl derivative and the imidazoline dicarboxyl coconut derivative; and cationic surfactants, such a cetyltrimethylammonium bromide.

When water, along with water-soluble emulifiers, are used in the compositions, it is necessary that they be used judiciously so that an aqueous foam is not formed when product is released from the container. An aqueous foam will neither produce nor sustain the required temperature when n-butane is used as the propellant. Including the water in the ointment in the form of a water-in-oil emulsion assures that an aqueous foam will not form.

Various therapeutic agents may also be included in the composition. These include local anesthetic ingredients such as benzocaine, dibucaine, lidocaine and pramoxine hydrochloride; antipruritic agents such as menthol and camphor; vasoconstrictors such as ephedrine sulfate, epinephrine and phenylephrine hydrochloride; antiseptics such as hexyl resorcinol, bithionol and triclocarban; antibiotics such as bacitracin, polymyxin, mystatin and neomycin; anti-inflammatory agents such as hydrocortisone; counter-irritants such as methyl salicylate; and rubefacients such as methyl nicotinate.

For the preparation of the compositions of this invention, ointments are prepared in the conventional manner. Generally, the ingredients are combined and heated with stirring until all ingredients have dissolved, except for those ingredients that are not soluble or are heat sensitive. These are added after the ointment has cooled sufficiently. The ointment is stirred while cooling. It is dosed into the aerosol containers at a temperature above its flow temperature.

When a aqueous phase is part of the ointment composition, ingredients that are soluble or dispersible in that phase are combined with it. Preferably, the aqueous phase is then blended with the non-aqueous phase at a temperature above the flow temperature of the non-aqueous phase to form a water-in oil emulsion. The two phases, either separately or as a performed emulsion, are dosed into the aerosol containers at a temperature above their flow temperatures.

Vacuum is applied to the containers to remove air and the propellant is added either before or after clinching of the valves. Either before or after adding the actuators and cover caps, the packages are passed through a water bath that is warm enough to raise the temperature of the ointment above its flow temperature. Shaking causes the ointment to blend with the propellant.

The studies that resulted in this invention were conducted using compositions packaged in aerosol containers fitted with valves with two 0.5 mm. diameter orifices and 3 mm. diameter dip tubes. The actuator had a spout with a 1 mm. diameter opening. About 2.5 grams of composition were expelled onto a paper held 2.5 cm. from the spout. The temperature was determined using an electronic thermometer inserted into the deposit. The paper holding the deposit was folded so that as much of the deposit as possible could surround the temperature probe. These test conditions were used in establishing the preferred temperature range and in determining how long the temperature was sustained.

To study various physical effects, actuators, valves and dip tubes with different size openings were used. Tests were also conducted when the distance between the actuator and the paper substrate were varied.

The following Examples 1-6 illustrate the preferred embodiments of the invention:

| Aerosol Ointment For Treatment Of Hemorrhoids | |
|---|---|
| Example 1 | Parts By Weight |
| Petroleum jelly (1) | 26.4 |
| Microcrystalline wax (2) | 6.6 |
| Epinephrine | 0.01 |
| Pramoxine hydrochloride | 1.0 |
| n-Butane | 66.0 |

| | |
|---|---|
| (1) flow temperature = 41°C | |
| (2) melting point = 74°C Ointment flow temperature = 48°C | |

This example illustrates a product for the relief of hemorrhoids. It was prepared by first milling the pramoxine hydrochloride with the petroleum jelly until the dispersion was complete. The dispersion was then combined with the wax and heated with stirring until the wax had dissolved in the petroleum jelly. The composition was then cooled to 55°C and the epinephrine mixed in. The fluid solution was added to the aerosol cans, valves were clinched on, vacuum was applied to remove air in the cans and n-butane was added under pressure. The filled cans were placed in a heated water bath to check for leaks and to bring the composition to a temperature above the flow temperature of the ointment. Spout actuators with a 1 mm. opening were placed on the valves and the cans were shaken to dissolve and/or disperse the ointment in the n-butane.

To use, the container was shaken and then held with the actuator close to a double layer of toilet tissue. About a 2 gram deposit was expelled onto the tissue. The ointment on the tissue was held against the hemorrhoids until it no longer felt cold. The sustained cold had the immediate effect of providing relief from burning and itching, while simultaneously the inflamed tissue appeared to shrink and recede to its normal position. These immediate beneficial effects due to the sustained cold were continued by the actions of the local anaesthetic and the vasoconstrictor present in the ointment.

| Aerosol Ointment For Treatment Of Sunburn | |
|---|---|
| Example | Parts By Weight |
| Glyceryl monostearate (1) | 11.6 |
| Isopropyl myristate | 17.4 |
| Camphor | 1.0 |
| n-Butane | 70.0 |

| | |
|---|---|
| (1) melting points = 57.4 C Ointment flow temperature = 44 C | |

Example 2 illustrates a sunburn preparation. It was prepared by heating with stirring to dissolve the glyceryl monostearate and camphor in the isopropyl myristate. The solution was cooled to 50°C and dosed into aerosol cans. Valves were crimped onto the cans, a vacuum was drawn and the propellant was added under pressure. The filled cans were placed in a heated water bath to check for leaks and to bring the composition above the flow temperature of the ointment. The cans were then shaken. The molten ointment mixes readily with the propellant in which it is dissolved and/or dispersed. Spray actuators with a 0.5 mm. diameter opening were fitted on the valves.

To use, the container was shaken and held only a few cm. from the sunburned area before spraying. The sustained cold quickly relieved burning and itching sensations due to the sunburn. The ointment was then spread to more uniformly cover the sunburned area. The antipruritic agent present in the composition continues the therapeutic effect.

| Aerosol Ointment For Treatment Of Arthritic Pain | |
|---|---|
| Example 3 | Parts By Weight |
| Glyceryl monostearate (1) | 17 |
| Methyl salicylate | 17 |
| n-Butane | 66 |

| | |
|---|---|
| (1) melting point = 57.5 C Ointment flow temperature - 38 C | |

This example illustrates a product for the relief of arthritic pain. The glyceryl monostearate and methyl salicylate were combined and heated to dissolve the glyceryl monostearate. The remainder of the procedure was the same as example 2, except that spout actuators with a 1 mm. diameter opening were used instead of spray actuators.

To use, the aerosol can was shaken and a small amount of the cold ointment was expelled and spread over the arthritic area. The sustained cold provided quick relief. The ointment was then rubbed into the area. As it was being rubbed in, the warm counterirritant action of the methyl salicylate could be felt through the cold.

| Aerosol Ointment For Treatment For Relieving Itching | |
|---|---|
| Example 4 | Parts By Weight |
| Glyceryl monostearate (1) | 10 |
| 2 Hexyldecanol | 15 |
| Hydrocortisone | 1 |
| n-Butane | 64 |
| Isobutane | 10 |

| | |
|---|---|
| (1) melting point = 57.5 C Ointment flow temperature = 40 C | |

The hydrocortisone preparation provides instant relief from itching. The glyceryl monostearate, 2-hexyldecanol and hydrocortisone were combined and heated with stirring to obtain a clear solution. The remainder of the procedure was the same as in example 2, except that spout actuators with a 1 mm. opening were used. When the aerosol ointment was applied, itching quickly stopped due to the sustained cold. The antipruritic effect continued throughout the day, presumable due to the action of the hydrocortisone.

| Aerosol Ointment For The Relief Of Itching | |
|---|---|
| Example 5 | Parts By Weight |
| Glyceryl monostearate (1) | 5.0 |
| Dimethylcyclopolysiloxane (2) | 12.0 |
| Hydrocortisone | 1.0 |
| Disodium cocoapmphodipropionate | 0.2 |
| Water | 15.8 |
| n-Butane | 66.0 |

| | |
|---|---|
| (1) melting point = 57.5 C | |
| (2) DC 245 Fluid (Dow Corning Corp.) Ointment flow temperature = 40 C | |

Example 5 illustrates a hydrocortisone ointment composition for the relief of itching, where the ointment component is a water-in-oil emulsion. It was prepared by combining the oil-soluble components, heating to dissolve the glyceryl monostearate, and then cooling with mixing until it started to thicken. The water-soluble surfactant was dissolved in the water and heated to the temperature of the oil mixture. The aqueous solution was mixed into the oil phase to form a water-in-oil emulsion, which was heated until it flowed, and then dosed into aerosol cans. The remainder of the procedure was the same as in Example 1.

| Aerosol Ointment For The Relief of Muscle Aches | |
|---|---|
| Example 6 | Parts By Weight |
| Glyceryl monostearate (1) | 8.6 |
| Dimethylcyclopolysiloxane (2) | 6.5 |
| Mineral oil | 6.5 |
| Menthol | 2.0 |
| Disodium cocoamphodipropionate | 0.15 |
| Water | 9.8 |
| n-Butane | 66.45 |

| | |
|---|---|
| (1) melting point = 57.5 C | |
| (2) DC 245 Fluid (Dow Corning Corp.) Ointment flow temperature = 41 C | |

Example 6 illustrates an ointment composition for the relief of muscle aches, where the ointment component is a water-in-oil emulsion. The procedure is the same as in Example 5.

The benefit derived from using compositions based on water-in-oil emulsions, especially when part of the oil phase is volatile, is that when applied topically the residue is not greasy or oily.

## Claims

1. An aerosol device comprising a therapeutic aerosol composition for topical use, where the aerosol composition consists of from 10 to 60 percent by weight of an ointment component and from 40 to 90 percent by weight of liquefied propellant that is predominantly a non-polar propellant and where the sum of the ointment component and the propellant equals 100 percent by weight of the composition, the composition when expelled from said aerosol device containing the composition being deposited as an ointment having a solid or semi-solid consistency and an initial temperature between - 5°C and +5°C, the ointment component being non-flowable below 35°C, said aerosol device comprising a valve and an actuator suitable for delivering said composition as said ointment.

2. An aerosol device as claimed in claim 1, wherein the ointment component comprises ingredients selected from the group consisting of oils, thickening agents, one or more of volatile organic liquids boiling below 250°C, and therapeutic agents wherein the oils, thickening agents and volatile organic liquids are dissolved and/or dispersed in the propellant.

3. An aerosol device as claimed in claim 1 or 2 wherein said ointment component comprises water and oil soluble emulsifiers.

4. An aerosol device as claimed in claim 2, wherein the ointment component comprises an aqueous phase comprising water, water-soluble emulsifying agents and optionally one or more therapeutic agents, wherein the aqueous phase when combined with the oil phase, forms a water-in-oil emulsion.

5. An aerosol device as claimed in any one of claims 1 to 4, where the liquefied propellant is n-butane alone or in combination with a lesser amount of higher vapour pressure propellant.

6. An aerosol device as claimed in claims 5, wherein said higher vapour pressure propellant is isobutane, propane or dimethyl ether.

7. An aerosol device as claimed in any one of claims 1 to 4, where the liquefied propellant is predominantly n-butane.

8. An aerosol device as claimed in claim 7, where the liquefied propellant additionally comprises isobutane.

9. An aerosol device as claimed in any one of claims 1 to 8 wherein the ointment component includes a therapeutic ingredient that provides relief from at least one of the pain, itching and discomfort of haemorrhoids, the pain and discomfort of arthritis, or the burning and discomfort of sunburn.

10. An aerosol device as claimed in any one of claims 2 to 9 comprising glyceryl monostearate as a thickening agent.

11. An aerosol device as claimed in any one of claims 1 to 10 wherein the ointment component comprises a therapeutic agent selected from the group consisting of an anti-inflammatory agent, a local anaesthetic, an analgesic, a counter-irritant, a vasoconstrictor, methyl salicylate or menthol.

12. An aerosol device as claimed in any one of claims 1 to 11 that, when expelled, forms a deposit which is of a solid or semi-solid nature, the deposit containing propellant whose evaporation is restrained by the solid or semi-solid nature of the deposit, thereby instantly upon topical application producing a sustained cooling effect.

13. An aerosol device as claimed in any one of claims 1 to 12 wherein the ointment component comprises oils selected from the group consisting of mineral oils, silicone oils, vegetable oils and synthetic oils, and thickening agents selected from the group consisting of mineral waxes, animal and vegetable waxes, synthetic waxes, lower alkylene hydrocarbon polymers and metallic soap.

14. An aerosol device as claimed in any one of claims 2 to 13 where the volatile organic liquid boiling below 250°C is a volatile silicone fluid.

15. An aerosol composition that enhances the therapeutic action of an ointment by instantly producing upon application to swollen, inflamed tissue a sustained cooling effect which provides fast relief from pain and itching as well as a tendency to shrink the swollen, inflamed tissue in advance of the slower action of any medication present in the ointment, where the aerosol composition consists essentially of 10 to 60 percent by weight of an ointment component that is non-flowable below 35°C and from 40 to 90 percent by weight of a liquefied propellant that is predominantly n-butane, and where the sum of the ointment component and the propellant equals 100 percent by weight, the composition when expelled from an aerosol device containing the composition depositing on the tissue, as a solid or semi-solid and at an initial temperature between -5°C and +5°C.

## Patentansprüche

1. Aerosolvorrichtung, umfassend eine therapeutische Aerosolzusammensetzung für eine topische Anwendung, wobei die Aerosolzusammensetzung aus 10 bis 60 Gewichtsprozent einer Salbenkomponente und aus 40 bis 90 Gewichtsprozent eines verflüssigten Treibmittels besteht, welches überwiegend ein nicht polares Treibmittel ist, und wobei die Summe aus der Salbenkomponente und dem Treibmittel 100 Gewichtsprozent der Zusammensetzung ergibt, wobei die Zusammensetzung, wenn sie aus der Aerosolvorrichtung, welche die Zusammensetzung enthält, ausgestoßen wird, als eine Salbe mit einer festen oder halbfesten Konsistenz und einer Anfangstemperatur zwischen -5°C und +5°C aufgetragen wird, wobei die Salbenkomponente unterhalb von 35°C nicht fließfähig ist, wobei die Aerosolvorrichtung ein Ventil und eine Betätigungsvorrichtung umfasst, welche geeignet ist für die Abgabe der Zusammensetzung als Salbe.

2. Aerosolvorrichtung nach Anspruch 1, worin die Salbenkomponente Bestandteile umfasst, welche ausgewählt sind aus der Gruppe bestehend aus Ölen, Verdickungsmitteln, einer oder mehreren flüchtigen organischen Flüssigkeiten, welche unterhalb von 250°C sieden, und therapeutischen Mitteln, worin die Öle, Verdickungsmittel, und flüchtigen organischen Flüssigkeiten im Treibmittel gelöst und/oder dispergiert sind.

3. Aerosolvorrichtung nach Anspruch 1 oder 2, worin die Salbenkomponente Wasser und öllösliche Emulgatoren umfasst.

4. Aerosolvorrichtung nach Anspruch 2, worin die Salbenkomponente eine wässrige Phase umfasst, welche Wasser, wasserlösliche Emulgiermittel und gegebenenfalls ein oder mehrere therapeutische Mittel umfasst, worin die wässrige Phase eine Wasser-in-Öl-Emulsion bildet, wenn sie mit der Ölphase vereinigt wird.

5. Aerosolvorrichtung nach einem der Ansprüche 1 bis 4, worin das verflüssigte Treibmittel n-Butan ist, alleine oder in Kombination mit einer geringeren Menge eines Treibmittels mit einem höheren Dampfdruck.

6. Aerosolvorrichtung nach Anspruch 5, worin das Treibmittel mit einem höheren Dampfdruck Isobutan, Propan oder Dimethylether ist.

7. Aerosolvorrichtung nach einem der Ansprüche 1 bis 4, worin das verflüssigte Treibmittel überwiegend n-Butan ist.

8. Aerosolvorrichtung nach Anspruch 7, worin das verflüssigte Treibmittel zusätzlich Isobutan umfasst.

9. Aerosolvorrichtung nach einem der Ansprüche 1 bis 9, worin die Salbenkomponente ein therapeutisches Mittel umfasst, welches Linderung schafft bei Schmerzen, Juckreiz und Beschwerden bei Hämorrhoiden, Schmerzen und Beschwerden bei Arthritis oder/und Verbrennungen und Beschwerden bei Sonnenbrand.

10. Aerosolvorrichtung nach einem der Ansprüche 2 bis 9, umfassend Glycerylmonostearat als Verdickungsmittel.

11. Aerosolvorrichtung nach einem der Ansprüche 1 bis 10, worin die Salbenkomponente ein therapeutisches Mittel umfasst, welches ausgewählt ist aus der Gruppe bestehend aus einem entzündungshemmenden Mittel, einem Lokalanästhetikum, einem Analgetikum, einem Gegenreizmittel, einem Vasokonstriktor, Methylsalicylat oder Methanol.

12. Aerosolvorrichtung nach einem der Ansprüche 1 bis 11, welche beim Ausstoßen eine Ablagerung bildet, welche eine feste oder halbfeste Beschaffenheit besitzt, wobei die Ablagerung ein Treibmittel enthält, dessen Verdampfung durch die feste oder halbfeste Beschaffenheit der Ablagerung behindert wird, wodurch sofort nach topischer Applikation eine anhaltende Kühlwirkung erzeugt wird.

13. Aerosolvorrichtung nach einem der Ansprüche 1 bis 12, worin die Salbenkomponente Öle umfasst, welche ausgewählt sind aus der Gruppe bestehend aus Mineralölen, Silikonölen, Pflanzenölen und synthetischen Ölen und worin die Verdickungsmittel ausgewählt sind aus der Gruppe bestehend aus Mineralwachsen, tierischen und pflanzlichen Wachsen, synthetischen Wachsen, niederen Alkylenkohlenwasserstoff-Polymeren und Metallseife.

14. Aerosolvorrichtung nach einem der Ansprüche 2 bis 13, worin die flüchtige, organische Flüssigkeit, welche unterhalb von 250°C siedet, ein flüchtiges Siliconfluid ist.

15. Aerosolzusammensetzung, welche die therapeutische Wirkung einer Salbe bei Applikation auf geschwollenes, entzündetes Gewebe durch sofortiges Erzeugen einer anhaltenden Kühlwirkung verstärkt, welche sowohl eine schnelle Linderung von Schmerzen und Juckreiz als auch eine Tendenz zum Abschwellen des geschwollenen, entzündeten Gewebes bereitstellt, vor der langsameren Wirkung eines in der Salbe vorliegenden Medikaments, wobei die Aerosolzusammensetzung im wesentlichen aus 10 bis 60 Gewichtsprozent einer Salbenkomponente, welche unterhalb von 35°C nicht fließfähig ist, und aus 40 bis 90 Gewichtsprozent eines verflüssigten Treibmittels, welches vorwiegend n-Butan ist, besteht, und wobei die Summe der Salbenkomponente und des Treibmittels 100 Gewichtsprozent ergibt, wobei die Zusammensetzung, wenn sie aus einer Aerosolvorrichtung, welche die Zusammensetzung enthält, auf das Gewebe aufgetragen wird, fest oder halbfest ist und eine Anfangstemperatur zwischen -5°C und +5°C besitzt.

## Revendications

1. Dispositif d'aérosol comprenant une composition thérapeutique en aérosol à l'usage topique, dans lequel la composition d'aérosol consiste en 10 à 60 pour-cent en poids d'un composant d'onguent et en 40 à 90 pour-cent en poids d'un agent propulseur liquéfié, qui est d'une manière prépondérante un agent propulseur non polaire et dans lequel la somme du constituant d'onguent et de l'agent propulseur représente 100 pour-cent du poids de la composition, la composition, lorsqu'elle est projetée du dispositif d'aérosol contenant la composition qui s'est déposée sous la forme d'un onguent, ayant une consistance solide ou semi-solide et une température initiale comprise entre -5°C et +5°C, le constituant d'onguent ne pouvant pas s'écouler en dessous de 35°C, le dispositif d'aérosol comprenant une valve et un actionneur approprié pour délivrer la composition sous la forme de l'onguent.

2. Dispositif d'aérosol tel que revendiqué à la revendication 1, dans lequel le constituant d'onguent comprend des ingrédients choisis dans le groupe consistant en des huiles, des agents épaississants, un ou plusieurs liquides organiques volatils dont le point d'ébullition est inférieur à 250°C, et des agents thérapeutiques, les huiles, les agents épaississants et les liquides organiques volatils étant dissous et/ou dispersés dans l'agent propulseur.

3. Dispositif d'aérosol tel que revendiqué à la revendication 1 ou 2, dans lequel le constituant d'onguent comprend de l'eau et des émulsionnants solubles dans l'huile.

4. Dispositif d'aérosol tel que revendiqué à la revendication 2, dans lequel le constituant d'onguent comprend une phase aqueuse comprenant de l'eau, des agents émulsionnants solubles dans l'eau et éventuellement un ou plusieurs agents thérapeutiques, la phase aqueuse lorsqu'elle est combinée à la phase huileuse formant une émulsion eau dans huile.

5. Dispositif d'aérosol tel que revendiqué à l'une quelconque des revendications 1 à 4, dans lequel l'agent propulseur liquéfié est du butane normal seul ou est combiné à une moindre quantité d'un agent propulseur ayant une tension de vapeur plus grande.

6. Dispositif d'aérosol tel que revendiqué à la revendication 5, dans lequel l'agent propulseur ayant une tension de vapeur plus grande est l'isobutane, le propane ou l'oxyde de diméthyle.

7. Dispositif d'aérosol tel que revendiqué à l'une quelconque des revendications 1 à 4, dans lequel l'agent propulseur liquéfié est d'une manière prépondérante du butane normal.

8. Dispositif d'aérosol tel que revendiqué à la revendication 7, dans lequel l'agent propulseur liquéfié comprend en outre de l'isobutane.

9. Dispositif d'aérosol tel que revendiqué à l'une quelconque des revendications 1 à 8, dans lequel le constituant d'onguent comprend un ingrédient thérapeutique qui soulage d'au moins l'un de la douleur, du prurit et du mal à l'aise dus aux hémorroïdes, de la douleur et du mal à l'aise dus à l'arthrite ou de la brûlure et du mal à l'aise de la brûlure solaire.

10. Dispositif d'aérosol tel que revendiqué à l'une quelconque des revendications 2 à 9, comprenant du monostéarate de glycéryle comme agent épaississant.

11. Dispositif d'aérosol tel que revendiqué à l'une quelconque des revendications 1 à 10, dans lequel le constituant d'onguent comprend un agent thérapeutique choisi dans le groupe consistant en un agent anti-inflammatoire, un anesthésique local, un analgésique, un calmant, un agent vasoconstricteur, le salicylate de méthyle ou le menthol.

12. Dispositif d'aérosol tel que revendiqué à l'une quelconque des revendications 1 à 11 qui, lorsqu'il y a projection, forme un dépôt qui est de nature solide ou semi-solide, le dépôt contenant l'agent propulseur dont l'évaporation est limitée par la nature solide ou semi-solide du dépôt, en produisant ainsi instantanément par application topique un effet de refroidissement soutenu.

13. Dispositif d'aérosol tel que revendiqué à l'une quelconque des revendications 1 à 12, dans lequel le constituant d'onguent comprend des huiles choisies dans le groupe consistant en des huiles minérales, des huiles de silicone, des huiles végétales et des huiles synthétiques, et des agents épaississants choisis dans le groupe consistant en des cires minérales, des cires animales et végétales, des cires synthétiques, des polymères hydrocarbonés d'alcoylène inférieur et du savon métallique.

14. Dispositif d'aérosol tel que revendiqué à l'une quelconque des revendications 2 à 13, dans lequel le liquide organique volatil bouillant en dessous de 250°C est un silicone fluide volatil.

15. Composition aérosol qui améliore l'effet thérapeutique d'un onguent en produisant instantanément, par application à des tissus gonflés et inflammés, un effet de refroidissement soutenu qui soulage rapidement de la douleur et du prurit, ainsi que de la tendance du tissu gonflé et inflammé à se rétracter, avant l'effet plus lent de toute médication présente dans l'onguent, la composition aérosol consistant essentiellement en 10 à 60 pour-cent en poids d'un constituant d'onguent qui ne peut pas s'écouler en dessous de 35°C et en 40 à 90 pour-cent en poids d'un agent propulseur liquéfié qui est d'une manière prépondérante du butane normal, et la somme du constituant d'onguent et de l'agent propulseur représentant 100 pour-cent en poids, la composition, lorsqu'elle est projetée d'un dispositif d'aérosol contenant la composition, se déposant sur les tissus sous la forme d'un solide ou d'un semi-solide et à une température initiale comprise entre -5°C et +5°C.
